# EUROPEAN PATENT SPECIFICATION

(11) **EP 1 552 308 B1**
(45) Date of publication and mention of the grant of the patent: **14.12.2011**
(21) Application number: 03772203.0
(22) Date of filing: 13.10.2003
(51) Int. Cl.: G01N 33/564, C07H 21/00, C12Q 1/68

(54) **DEMETHYLATED AND/OR OXIDIZED MEMBRANE DNA AND USE THEREOF IN THE DIAGNOSTIC OF AUTOIMMUNE DISEASES**
ENTMETHYLIERTE UND/ODER OXIDIERTE MEMBRAN DNS UND DEREN VERWENDUNG ZUR DIAGNOSE VON AUTOIMMUNKRANKHEITEN
ADN MEMBRANAIRE DEMETHYLE ET/OU OXYDE ET SON UTILISATION DANS LE DIAGNOSTIC DE MALADIES AUTO-IMMUNES

(30) Priority: 15.10.2002 US 270160; 15.01.2003 EP 03000837
(43) Date of publication of application: 13.07.2005
(73) Proprietor: BIOTECH TOOLS S.A., 1120 Bruxelles (BE)
(72) Inventor: LEGON, Thierry, B-3360 Korbeek Lo (BE); HENOT, Frédéric, B-1040 Bruxelles (BE); QUESTIAUX, Nadine, B-1350 Orp-le-Grand (BE)
(74) Representative: von Kreisler Selting Werner
(86) International application number: PCT/EP2003/011293
(87) International publication number: WO 2004/036219

(56) References cited:
- US-A- 6 057 097
- SERVAIS GENEVIEVE ET AL: "Diagnostic specificities and sensitivities of anti dsDNA, anti membrane DNA and anti nucleosomes autoantibodies." SCANDINAVIAN JOURNAL OF CLINICAL AND LABORATORY INVESTIGATION, vol. 61, no. Supplement 235, 2001, pages 61-67, XP009008282 ISSN: 0036-5513
- SERVAIS GENEVIEVE ET AL: "Evidence of autoantibodies to cell membrane associated DNA (cultured lymphocytes): A new specific marker for rapid identification of systemic lupus erythematosus." ANNALS OF THE RHEUMATIC DISEASES, vol. 57, no. 10, October 1998 (1998-10), pages 606-613, XP009008284 ISSN: 0003-4967
- FRENKEL KRYSTYNA ET AL: "Recognition of oxidized DNA bases by sera of patients with inflammatory diseases." FREE RADICAL BIOLOGY & MEDICINE, vol. 14, no. 5, 1993, pages 483-494, XP009008279 ISSN: 0891-5849
- FRENKEL K ET AL: "Enhanced antibody titers to an oxidized DNA base in inflammatory and neoplastic diseases." EXPERIMENTAL DERMATOLOGY. DENMARK DEC 1992, vol. 1, no. 5, December 1992 (1992-12), pages 242-247, XP009008280 ISSN: 0906-6705

## Description

### Field of the invention

The present invention relates to a process for the preparation of oxidized and/or demethylated cytoplasmic membrane-associated DNA (cmDNA) or membrane DNA (mDNA) and oxidized and/or demethylated mDNA.

It further relates to a cell comprising the oxidized and/or demethylated membrane DNA and the diagnostic and pharmaceutical use of the oxidized and/or demethylated membrane DNA and the cells comprising such mDNA.

### Background of the invention

US 6,057,097, incorporated per reference, disclosed markers for pathologies comprising an autoimmune reaction and markers for inflammatory diseases.

As disclosed in this document, numerous pathologies comprising an autoimmune reaction and inflammatory diseases have an uncertain or unknown etiology and may have a multifactor origin.

The diagnosis of some of these diseases is difficult or uncertain. One disease especially mentioned in this document is systemic lupus erythematosus (SLE). Prior to US 6,057,097 it had not been possible to provide a sufficient specific antigenic structure to obtain a reliable diagnosis both in specificity and sensitivity for the mentioned pathologies, especially SLE.

US 6,057,097 solves the problem by providing an antigenic structure named cytoplasmic-membrane associated DNA (cmDNA) or membrane DNA (mDNA).

This antigen is recognized by antibodies present in biological fluids of subjects suffering from the mentioned diseases. This antigenic structure is prepared from cells especially from B-lymphocytes such as Wil-2 cells.

It is the aim of the present invention to provide an improved antigenic structure and thereby an improved diagnostic tool showing increased specificity and sensibility :

A further aim is to provide a screening method for pharmaceutical substances useful in the treatment of autoimmune diseases and/or inflammations.

A further aim is to provide a pharmaceutical composition.

### Summary of the invention

According to the invention, the antigenic structure is prepared by a process for the preparation of membrane DNA in an oxidized and/or demethylated condition.

This process comprises the steps of
- treating a cell with a stress factor selected from the group consisting of UV-irradiation, oxidizing reagents, heavy metal salts, drugs, nucleoside and nucleotide analogs, enzyme inhibitors, enzyme activators and pH-shift
- lyses of the cell to give a cell lysate
- purification of oxidized and/or demethylated mDNA from the cell lysate.

It is believed that treatment with stress factors increases the amount of oxidized membrane DNA and/or increases demethylation of membrane DNA in the cell. Afterwards, the mDNA is isolated in a similar manner as previously known from US 6,057,097.

"Oxidized and/or demethylated mDNA" is mDNA that is either oxidized, or demethylated or broth.

"Oxidized membrane DNA" is membrane DNA comprising more oxidized nucleotides in relation to mDNA from cells not treated with stress factors. Said oxidized nucleotides can be for example either 5-hydroxymethyl-2'-deoxyuridine, and/or thymidine glycol, and/or 7,8-dihydrox-8-oxoadenosine and/or 8-oxo-7,8-dihydro-2'-deoxyguanosine.

The most abundant oxidized nucleotide is 8-oxo-7,8-dihydro-2'-deoxyguanosine (8-oxo-dG). The oxidized mDNA may comprise preferably more than 0.5%, more preferred more than 1%, most preferred more than 3% of all guanosine in an oxidized form.

"Demethylated mDNA" is an mDNA comprising less methylated nucleotides than DNA prepared from the cells without treatment with a stress factor.

The methylated nucleotide is usually cytosine. Such methylation mostly occurs in the context of CpG dinucleotides in vertebrates and is often associated with transcriptional repression.

Preferable the amount of 5-methylated cytosine is less than 90% of untreated mDNA, more preferably 50% or less and more preferred 30% or less.

It is also believed that treatment with stress factors, such as but not limited to oxidative stress, may generate transversions or mutations, such as but not limited to G to T, in the sequence of membrane DNA in the cell. Hence, "oxidized and/or demethylated mDNA" can be membrane DNA comprising at least the same amounts of oxidized and/or methylated nucleotides in relation to mDNA from cells not treated with stress factors, but with a different nucleotidic sequence.

Suitable stress factors are for example dichromate, hydrogen peroxide, permanganate, quinidine, D-pencillamine, Hydralazine, procainamide, RNA and/or DNA metabolites, nucleoside and nucleotide analogs such as 5-azacytidine, 5-aza-2'-deoxycytidine, inhibitors of DNA methylases, inhibitors of histone deacetylases such as butyrates or trichostatin A, inhibitors of histone acetyltransferases, inhibitors of histone arginine and histone lysine methyltransferases, inhibitors of protein kinases such as staurosporine or calphostin C or K-252a or H-89, activators of protein kinases such as phorbol esters or bryostatin 1, inhibitors of protein phosphatases such as calyculin A or okadaic acid, inhibitors of poly(ADP-ribose) polymerase such as 3-aminobenzamide or m-Iodobenzylguanidine hemisulfate, inhibitors of ubiquitin conjugating enzymes such as methylated ubiquitin, inhibitors of ubiquitin C-terminal hydrolase such as ubiquitin aldehyde, inhibitors of enzymes involved in correction of replicational errors and/or spontaneous DNA damage such as cadmium ions, inhibitors of N-glycosylation such as tunicamycin, inhibitors of 20S or 26S proteasomes such as lactacystin, inhibitors of farnesylation such as alpha-hydroxy-farnesylphophonic- acid, inhibitors of geranylgeranylation, inhibitors of protein methylation such as ebelactone B, inhibitors of catalase, inhibitors of superoxide dismutase, inhibitors of glutathione peroxidase, inducers of DNA hypermethylation, inducers of phospholipid methylation, inhibitors of phospholipid methylation such as 3-deazaadenosine or 3-deaza-(±)-aristeromycin, or combinations thereof.

A further suitable stress factor is a pH-shift. A pH-shift is a change of the pH of the environment of at least 0.2 pH-units, preferably 0.4 pH-units.

A further suitable stress factors is for example UV irradiation. UV irradiation is preferably combined with oxidizing reagents.

Suitable oxidizing reagents are for example H₂O₂ or molecular oxygen.

From molecular oxygen, active species such as superoxide radical (O₂⁻•) and hydroxyl radical - (HO•) can be obtained, see for example "proceedings of the society for experimental biology and medicine" 1999, pages 246 to 252.

Suitable heavy metal salts are cadmium salts, chrome salts or mixtures thereof. They can be used alone or in combination with the oxidizing reagents for example O₂ or H₂O₂.

A further embodiment of the present invention is the oxidized and/or demethylated membrane DNA (mDNA) obtainable according to the method of the invention.

Such oxidized and/or demethylated membrane DNA or fragments thereof are useful as an antigenic structure in a diagnostic test of autoimmune diseases. Fragments of mDNA or fragments of oxidized and/or demethylated mDNA can be obtained by methods known in the art such as, but not limited to, sonication, enzymatic digestion with nucleases and/or restriction enzymes.

In a further embodiment the oxidized and/or demethylated mDNA or fragments thereof are further associated with DNA binding proteins, most preferably, with histones.

These DNA binding proteins may be hypo- or hyperacetylated, hypo- or hyperphosphorylated, hypo- or hypermethylated, hypo- or hyperubiquitinated, hypo- or hyper-(poly-ADP-ribosylated) or hypo- or hyperglycosylated or combinations thereof.

A further embodiment of the present invention are cells comprising membrane DNA of the present invention.

In a preferred embodiment of the invention, the cells are selected from the group of leucocytes, especially neutrophils, and/or B-lymphocytes and/or lymphoblastoid cells and/or monocytes, or the cells are selected from the groups of microorganisms, such as but not limited to Tetrahymena thermophila and Tetrahymena pyriformis.

Such cells can be obtained by a process comprising the step of treating a cell with a stress factor selected from the group consisting of UV-irradiation, oxidizing reagents, heavy metal salts, drugs, nucleoside and nucleotide analogs, enzyme inhibitors, enzyme activators and pH-shift.

Suitable stress factors are disclosed supra.

A further embodiment of the invention is a diagnostic agent comprising oxidized and/or demethylated membrane DNA, fragments of this mDNA or cells comprising the oxidized and/or demethylated membrane DNA.

This diagnostic agent can be used in different diagnostic tests.

In one embodiment, the present invention covers therefore a process for the detection of an autoimmune disease comprising the steps of
- bringing a biological fluid of an animal containing antibodies into contact with the diagnostic agent of the invention.
- measuring binding of said antibodies with said diagnostic agent
wherein binding indicates an autoimmune disease of the animal.

In a preferred embodiment, the diagnostic agent is in the form of cells and the cells are or may be attached to a solid support prior to bringing the biological fluid in contact with the diagnostic agent. Such an attachment may be covalently or not covalently, either through ligand/receptor or ligand/protein interaction or biotinylated cells/(strept)avidin, lectin/anti-lectin or through a covalent binding between an activated solid support and amino, acidic or sulfhydryl groups or carbohydrates or oxidized carbohydrates of the cell surface.

In a second embodiment, the present invention covers a process for the in-vitro detection of an autoimmune disease comprising the steps of
- bringing a biological sample of an animal containing cells into contact with antibodies directed against the oxidized and/or demethylated mDNA or fragment thereof of claim 4
- measuring binding of said antibodies with said cells
wherein binding indicates a autoimmune disease of the animal.

Preferably these diagnostic tests are done *in-vitro.*

Such diagnostic tests are also useful with other antigens, that are (bio)chemically modified by treating a cell with a stress factor.

In a suitable test, the cells could be used to identify binding of antibodies from the biological fluids, especially blood or sera to the cells.

Alternatively, the oxidized and/or demethylated mDNA or fragments thereof could be used in a test system, for example in an ELISA or in a gel shift assay to identify binding.

It is a further embodiment of the invention to use the diagnostic agent to detect binding of antibodies to membrane DNA. Such a binding indicates an autoimmune disease of the animal.

In a further embodiment, the present invention provides a method for identifying drugs by a screening method. This screening method comprises measuring the binding of antibodies to the oxidized and/or demethylated mDNA or fragments thereof or cells comprising oxidized and/or demethylated mDNA. The influence of potential substances on the binding of the antibodies indicates whether or not such substances are useful pharmaceutical compositions.

Yet another embodiment of the present invention is a pharmaceutical formulation comprising the oxidized and/or demethylated mDNA or fragments thereof or cells comprising oxidized and/or demethylated DNA. Such a pharmaceutical composition could be used to detract autoimmune antibodies of a patient from structures of the patient, thereby reducing or healing the disease.

This pharmaceutical formulation could also be used in form of a vaccine.

Therefore, the use of the pharmaceutical formulation for treating autoimmune diseases in animal is also part of the invention.

The present invention is further explained by the following, non-limiting examples.

### Examples

### Cell culture

The cell line of human lymphoblastoid B (Wil2 NS) obtained from ICN Flow Laboratories (ECACC No 90112121) is maintained in RPMI 1640 supplemented with 10% foetal bovine serum (heat inactivated and tested for the absence of mycoplasma), L-glutamine and 1% penicillin-streptomycin, in an humid oven at 37°C and 5% CO₂,

### Sera

The sera are obtained from patients affected by several inflammatory diseases or pathologies having an auto-immune reaction and from normal individuals. The sera are obtained from centrifuged coagulated blood and held at -20°C until use.

In all the experiments described below, sera of SLE patients that are positive in the test according to US 6,057,97 are always positive whatever the cell treatment is.

LED 0 means SLE patients, having at least four of the SLEDAI criteria, of whom sera diluted 1/30 do not give a positive pattern in the test of US 6,057,097.

RF is used for "sera from patients affected by several inflammatory diseases or pathologies but not SLE".

### In-vitro oxidation of mDNA

Wil2 NS cells are washed three times with Hank's solution (Gibco BRL) and re-suspended at 0.25x10⁶ cells/mL in HBSS and subsequently spotted on glass slides divided into 20 µL/wells. After drying at 37°C during 2 hours, in an oven, the cells are fixed for 3 minutes in methanol. The cells are then incubated for 30 minutes at room temperature in the presence of goat anti-human albumin serum (Dia-Sorin) diluted at 1/35 in 30 µL PBS. Slides are then washed in PBS.

The slides were exposed to 1% of hydrogen peroxide in PBS and immediately irradiated with UVB at 254 nm during 1 hour under a UV-CAMAG lamp. Irradiation was performed at room temperature in the dark at 13 cm of the slides.

The slides are incubated for 30 minutes in the presence of different patients sera diluted 1/10 in PBS/Tween 0.05% (cell culture grade) at 20 µl / well and washed again with PBS. The slides are further incubated for 30 minutes in the dark in the presence of 30 µL of a fluorescein-conjugated goat anti-human IgG (Inova) dialyzed overnight at 4°C in Slide-A-Lyser 10,000 MWCO (Pierce) and then centrifuged three times on a Centricon MY-10 (Millipore). A final wash of the slides is performed with PBS alone, followed by PBS containing Evans Blue as counterstain. Finally, slides are mounted in glycerin/PBS (1:1) pH 8.4, and visualized by means of UV immersion (Nikon).

### Results :

In-vitro oxidation was performed with H₂O₂+UV₂₅₄ₙₘ, and 45 sera were tested (5 healthy subjects, 10 auto-immuns, and 30 rheumatoid arthritis).

| Sera, dilution 1/10 | No oxidation US 6,057,097 | | oxidation | |
|---|---|---|---|---|
| | positive | negative | positive | negative |
| LED 0 | 8 | 2 | 7 | 3 |
| RF | 32 | 13 | 3 | 42 |

Obviously, in-vitro oxidation of mDNA increases specificity and sensibility of the test, and allows to identify more SLE patients.

### In-vivo oxidation of mDNA

A pool of cadmium acetate (Cd), sodium dichromate (Cr) and hydrogen peroxide (H₂O₂) was added (at the final concentrations of 10 µM, 10 µM and 20 µM respectively) to the culture 17 hours 30 minutes prior to the removing of cells in the growing logarithmic phase. The preparation, at a density of 1.0 to 1.5x10⁶ cells/mL, was assessed for viability both before and at the end of the experiment by trypan blue exclusion. Viability must be more than 95%.

Wil2 NS cells are washed once in PBS (Phosphate buffered saline, 10 mM, pH 7.4), then once in PBS supplemented with ATP at a final concentration of 500 µM. After that, they are incubated for 30 minutes at room temperature in the presence of goat anti-human albumin (Dia-Sorin) diluted at 1/35 in PBS. Cells are then collected by centrifugation, and re-suspended at 0.26×10⁶ cells/mL and subsequently spotted on glass slides divided into 20 µL/wells,

After drying at 37°C during 2 hours in an oven, the cells are fixed for 3 minutes in methanol and washed once in PBS (Phosphate buffered Saline, 10 mM, pH 7.4).

They are incubated for 30 minutes in the presence of different patients sera diluted 1/20 in PBS/Tween 0.05% (cell culture grade) at 20 µL / well and washed again with PBS. The slides are further incubated for 30 minutes in the dark in the presence of 30 µL of a mixture of fluorescein-conjugated goat F(ab₂)' anti-human IgG (Sigma, dilution 1/60) and fluorescein-conjugated goat IgG anti-human IgM (Sigma, dilution 1/70). A final wash of the slides is performed with PBS alone, followed by PBS containing Evans Blue as counterstain. Finally, slides are mounted in glycerin/PBS (1:1), pH 8.4, and visualized by means of UV immersion microscope (Nikon), magnification (40X).

### Results:

When cells growth occurs in RPMI 1640 supplemented with 10% FBS and the various products, only 10 of 327 sera from patients affected by several inflammatory diseases or pathologies but not SLE, give a positive pattern, and 15 of 31 sera referenced as "LED 0" give a positive pattern. The test has a specificity of 97% and a sensibility of 82%.

When cells growth occurs in a serum-free medium (AIMV, Life Technologies) and the various products, only 6 of 347 sera from patients affected by several inflammatory diseases or pathologies but not SLE, give a positive pattern, and 18 of 31 sera referenced as "LED 0" give a positive pattern. The test has a specificity of 98.3% and a sensibility of 84%.

### In-vivo inhibition of DNA_methylases

5-Aza-cytidine was added at the final concentrations of 1.0 µM to the culture 17 hours 30 minutes prior to the removing of cells in the growing logarithmic phase. The preparation, at a density of 1.5 to 1.8 to 2.0×10⁶ cells/ml, was assessed for viability both before and at the end of the experiment by trypan Blue exclusion. Viability must be more than 95%.

The slides are incubated for 30 minutes in the presence of different patients sera diluted 1/20 in PBS/Tween 0.05% (cell culture grade) at 20 µL/well and washed again with PBS.

The slides are further incubated for 30 minutes in the dark in the presence of 30 µl of a mixture of fluorescein -conjugated goat F(ab₂)' anti-human IgG (Sigma, dilution 1/60) and fluorescein-conjugated goat IgG anti-human IgM (Sigma, dilution 1/70).

### Results:

| sera | quantity | dilution | Ox | AzaC |
|---|---|---|---|---|
| LED 0 | 3 | 1/10 | 3 | 3 |
| RF | 7 | 1/10 | 7 | 2 |

| | | | | |
|---|---|---|---|---|
| ox: Cr+Cd+H₂O₂; AzaC: 5-aza-cytidine. | | | | |

Demethylated mDNA is also a good antigen and is well recognized by anti-mDNA antibodies of SLE patients sera. Specify is increased a well as sensibility because sera can be diluted 1/10 instead of 1/30.

It should be noticed here that 6 of the 7 RF sera are those that were "positive" in the experiment described above.

### In-vivo inhibition of histone deacetylation

Sodium butyrate was added at the final concentrations of 1.0 mM to the culture medium (serum-free medium AIMV, Life Technologies) 17 hours 30 minutes prior to the removing of cells in the growing logarithmic phase. The preparation, at a density of 1.5 to 1.8x10⁶ cells/ml, was assessed for viability both before and at the end of the experiment by trypan Blue exclusion.

### Viability must be more than 95%

The slides are incubated for 30 minutes in the presence of different patients sera diluted 1/20 in PBS/Tween 0.05% (cell culture grade) at 20 µL/well and washed again with PBS.

The slides am further incubated for 30 minutes in the dark in the presence of 30 µl of a mixture of fluorescein-conjugated goat F(ab₂)' anti-human IgG (Sigma, dilution 1/60) and goat IgG anti-human IgM (Sigma, dilution 1/70).

### Results:

| sera | quantity | dilution | NaBut |
|---|---|---|---|
| LED 0 | 7 | 1/20 | 7 |
| RF | 15 | 1/20 | 1 |

| | | | |
|---|---|---|---|
| NaBut: sodium butyrate | | | |

It seems that controlling histone hyperacetylation improves the selectivity of the test. Hence, membrane DNA might be associated with hyperacetylated histones on the cell surface (nucleosome-like structures)

### Use of drugs

Hydralazine or procainamide were added at the final concentration of 10⁻⁵ M to the culture medium (serum-free medium AIMV, Life Technologies) 17 hours 30 minutes prior to the removing of cells in the growing logarithmic phase. The preparation, at a density of 1.5 to 1.8x10⁶ cells/ml, was assessed for viability both before and at the end of the experiment by trypan Blue exclusion.

### Viability must be more than 95%

The slides are incubated for 30 minutes in the presence of different patients sera diluted 1/20 in PBS/Tween 0.05% (cell culture grade) at 20 µL/well and washed again with PBS.

The slides are further incubated for 30 minutes in the dark in the presence of 30 µl of a mixture of fluorescein-conjugated goat F(ab₂)' anti-human IgG (Sigma, dilution 1/60) and goat IgG anti-human IgM (Sigma, dilution 1/70).

### Results:

| sera | quantity | dilution | Hydralazine | Procainamide |
|---|---|---|---|---|
| LED 0 | 6 | 1/20 | 2 | 2 |
| RF | 9 | 1/20 | 2 | 1 |

After treating the cells with one of the drugs, well-known to induce lupus, mDNA is also a good antigen and is well recognized by anti-mDNA antibodies of SLE patients sera. Specifity is increased a well as sensibility because sera can be diluted 1/20 instead of 1/30 or 1/40.

### Presence-of 8-oxo-2'-deoxyguanosine

The H₂O₂-UV irradiated slides or slides produced with cells grown in presence of either Cd+Cr+H₂O₂ or 5-azacytidine, or sodium butyrate are incubated overnight at 4°C in the presence of 30 µl of an anti-8-oxo-dG mouse monoclonal antibody (Gentaur) diluted 1/100 in 10 mM Tris.HCl, pH 7.5, 10% Foetal Bovine Serum. The slides are washed once in PBS and then incubated for 40 minutes in the presence of a goat anti-mouse IgG1 antibody (southern Biotechnology Associates, Inc) diluted at a concentration of 0.1 µg / 1×10⁶ cells in PBS.

A final wash of the slides is performed in PBS and slides are mounted in glycerin/PBS pH 8.4 prior visualization by means of UV immersion microscope.

### Results:

A positive pattern 5 represented by a cell membrane green punctuate pattern. 8-Oxo-dG is shown to be present at the cell surface when oxidation of mDNA was performed either *in-vivo* (Cd+Cr+H₂O₂) or *in-vitro* (H₂O₂+UV) according to the method described supra.

A positive pattern is also shown when cells were grown in the presence of 5-aza-cytidine but not in presence of sodium butyrate.

### Presence of 5-methylcytosine

The slides produced with cells grown in presence of either Cd+Cr+H₂O₂ or 5-azacytidine, or sodium butyrate are incubated overnight at 4°C in the presence of 30 µl of an anti-5 methylcytosine sheep antibody (Abcam, Ab1884) diluted 1/100 in PBS. The slides are washed once in PBS and then incubated for 15 seconds in the presence of 30 µl of NaOH 7×10⁻⁵ N. After washing with PBS, the slides are finally incubated 30 minutes in the presence of a donkey anti-sheep IgG antibody (Southern Biotechnology Associates, Inc) diluted at a concentration of 0.1 µg/1×10⁶ cells in PBS.

A final wash of the slides is performed In PBS and slides are mounted in glycerin/PBS pH 8.4 prior visualization by means of UV immersion microscope.

### Results:

A positive pattern to identify 5-methylcytosine within the cells is represented by an intracellular green fluorescence.

Strong overall methylation of DNA occurred for cells grown in presence of either Cd+Cr+H₂O₂ or sodium butyrate but not in the presence of 5-azacytidine, even if intracellular green fluorescence of cells treated by sodium butyrate is not as strong as the first case.

## Claims

1. A process for the preparation of oxidized and/or demethylated membrane DNA (mDNA) comprising the steps of
• treating a cell with a stress factor selected from the group consisting of UV-irradiation, oxidizing reagents, heavy metal salts, drugs, nucleoside and nucleotide analogs, enzyme inhibitors and pH-shift
• lyses of the cell to give a cell lysate
• purification of oxidized and/or demethylated mDNA from the cell lysate.

2. The process of claim 1, wherein the stressfactor is selected from dichromate, hydrogen peroxide, permanganate, quinidine, D-pencillamine, hydralazine, procainamide, RNA/DNA metabolites, nucleoside and nucleotide analogs such as 5-aza-cytidine, 5-aza-2'-deoxycytidine, inhibitors of DNA methylases, inhibitors of histone deacetylases such as butyrates or trichostatin A, inhibitors of histone acetyltransferases, inhibitors of histone arginine and histone lysine methyltransferases, inhibitors of protein kinases such as staurosporine or calphostin C or K-252a or H-89, activators of protein kinases such as phorbol esters or bryostatin 1, inhibitors of protein phosphatases such as calyculin A or okadaic acid, inhibitors of poly(ADP-ribose) polymerase such as 3-aminobenzamide or m-Iodobenzylguanidine hemisulfate, inhibitors of ubiquitin conjugating enzymes such as methylated ubiquitin, inhibitors of ubiquitin C-terminal hydrolase such as ubiquitin aldehyde, inhibitors of enzymes involved in correction of replicational errors and/or spontaneous DNA damage such as cadmium ions, inhibitors of N-glycosylation such as tunicamycin, inhibitors of 20S or 26S proteasomes such as lactacystin, inhibitors of farnesylation such as alpha-hydroxy-farnesylphosphonic acid, inhibitors of geranylgeranylation, inhibitors of protein methylation such as ebelactone B, inhibitors of catalase, inhibitors of superoxide dismutase, inhibitors of glutathione peroxidase, inducers of DNA hypermethylation, inducers of phospholipid methylation, inhibitors of phospholipid methylation such as 3-deazaadenosine or 3-deaza-(±)-aristeromycin, or combinations thereof.

3. The process of claim 1 wherein the oxidized and/or demethylated mDNA is associated with nucleic acid binding proteins.

4. Oxidized and/or demethylated membrane DNA (mDNA) obtainable according to the method of any one of claims 1 to 3.

5. mDNA according to claim 4 associated with DNA-binding proteins, especially histones.

6. mDNA according to claim 5, wherein the DNA-binding proteins are hypo- or hyperacetylated and/or hypo- or hyperphosphorylated, and/or hypo- or hypermethylated, and/or hypo- or hyper-ubiquitinated, and/or hypo- or hyper-(poly-ADP-ribosylated), and/or hypo- or hyperglycosylated or combinations thereof.

7. A diagnostic agent comprising the mDNA according to any one of the claims 4 to 6 or cells obtainable by a process for preparing cells comprising oxidized and/or demethylated membrane DNA comprising the step of treating a cell with a stress factor selected from the group consisting of UV-irradiation, oxidizing reagents, heavy metal salts, drugs, nucleoside and nucleotide analogs, enzyme inhibitors, enzyme activators and pH-shift, wherein the treatment with stress factors increases the amount of oxidized membrane DNA and/or increases demethylation of membrane DNA in the cell.

8. A process for the in-vitro detection of systemic lupus erythematosus **(SLE)** comprising the steps of
• bringing a biological fluid of an animal containing antibodies into contact with the diagnostic agent of claim 7
• measuring binding of said antibodies with said diagnostic agent wherein binding indicates a disease of the animal.

9. A process for the in-vitro detection of **systemic lupus erythematosus (SLE)** comprising the steps of
• bringing a biological sample of an animal containing cells into contact with antibodies directed against the oxidized and/or demethylated mDNA of claim 4
• measuring binding of said antibodies with said cells
wherein binding indicates a disease of the animal.

10. Use of the diagnostic agent of claim 7 to detect binding of antibodies to mDNA in-vitro.

11. Use according to claim 10, wherein binding of antibodies from an animal indicates an autoimmune disease of the animal.

12. A method for screening drugs by measuring the binding of antibodies to the mDNA according to any one the claims 4 to 6 in the presence of a drug.

13. A pharmaceutical formulation comprising the mDNA according to any one the claims 4 to 6.

14. The pharmaceutical formulation of claim 13 in form of a vaccine.

15. Use of the mDNA according to anyone of claims 4 to 6 for the preparation of a medicament for treating autoimmune diseases in an animal.

## Patentansprüche

1. Verfahren zur Herstellung von oxidierter und/oder demethylierter Membran-DNA (mDNA), das die folgenden Schritte umfasst:
• Behandeln einer Zelle mit einem Stressfaktor, der aus der Gruppe ausgewählt ist, die aus UV-Bestrahlung, Oxidationsmitteln, Schwermetallsalzen, Wirkstoffen, Nucleosid- und Nucleotid-Analoga, Enzyminhibitoren und pH-Verschiebungen besteht;
• Lyse der Zelle unter Bildung eines Zelllysats;
• Reinigung von oxidierter und/oder demethylierter mDNA aus dem Zelllysat.

2. Verfahren gemäß Anspruch 1, wobei der Stressfaktor ausgewählt ist aus Dichromat, Wasserstoffperoxid, Permanganat, Chinidin, D-Penicillamin, Hydralazin, Procainamid, RNA/DNA-Metaboliten, Nucleosid- und Nucleotid-Analoga, wie 5-Azacytidin, 5-Aza-2-desoxycytidin, Inhibitoren von DNA-Methylasen, Inhibitoren von Histon-Deacetylasen, wie Butyraten oder Trichostatin A, Inhibitoren von Histon-Acetyltransferasen, Inhibitoren von Histon-Arginin- und Histon-Lysin-Methyltransferasen, Inhibitoren von Protein-Kinasen, wie Staurosporin oder Calphostin C oder K-252a oder H-89, Aktivatoren von Protein-Kinasen, wie Phorbolester oder Bryostatin 1, Inhibitoren von Protein-Phosphatasen, wie Calyculin A oder Okadainsäure, Inhibitoren von Poly(ADP-Ribose)-Polymerase, wie 3-Aminobenzamid oder m-Iodbenzylguanidin-Hemisulfat, Inhibitoren von Ubiquitin-konjugierenden Enzymen, wie methyliertes Ubiquitin, Inhibitoren von Ubiquitin-C-Terminus-Hydrolase, wie Ubiquitinaldehyd, Inhibitoren von Enzymen, die an der Korrektur von Replikationsfehlern und/oder spontanen DNA-Schäden beteiligt sind, wie Cadmium-Ionen, Inhibitoren der N-Glycosylierung, wie Tunicamycin, Inhibitoren von 20S- oder 26S-Proteasomen, wie Lactacystin, Inhibitoren der Farnesylierung, wie alpha-Hydroxyfarnesylphosphonsäure, Inhibitoren der Geranylgeranylierung, Inhibitoren der Proteinmethylierung, wie Ebelacton B, Inhibitoren von Katalase, Inhibitoren von Superoxid-Dismutase, Inhibitoren von Glutathion-Peroxidase, Induktoren der DNA-Hypermethylierung, Induktoren der Phospholipid-Methylierung, Inhibitoren der Phospholipid-Methylierung, wie 3-Desazaadenosin oder 3-Desaza-(±)-aristeromycin, oder Kombinationen davon.

3. Verfahren gemäß Anspruch 1, wobei die oxidierte und/oder demethylierte mDNA mit Nucleinsäure-bindenden Proteinen assoziiert ist.

4. Oxidierte und/oder demethylierte Membran-DNA (mDNA), die nach dem Verfahren gemäß einem der Ansprüche 1 bis 3 erhältlich ist.

5. mDNA gemäß Anspruch 4, die mit DNA-bindenden Proteinen, insbesondere Histonen, assoziiert ist.

6. mDNA gemäß Anspruch 5, wobei die DNA-bindenden Proteine hypo- oder hyperacetyliert und/oder hypo- oder hyperphosphoryliert und/oder hypo-oder hypermethyliert und/oder hypo- oder hyperubiquitiniert und/oder hypo- oder hyper(poly-ADP-ribosyliert) und/oder hypo- oder hyperglycosyliert oder Kombinationen davon sind.

7. Diagnostisches Mittel, umfassend die mDNA gemäß einem der Ansprüche 4 bis 6 oder Zellen, die erhältlich sind durch ein Verfahren zur Herstellung von Zellen, die oxidierte und/oder demethylierte Membran-DNA umfassen, das den Schritt des Behandelns einer Zelle mit einem Stressfaktor umfasst, der aus der Gruppe ausgewählt ist, die aus UV-Bestrahlung, Oxidationsmitteln, Schwermetallsalzen, Wirkstoffen, Nucleosid- und Nucleotid-Analoga, Enzyminhibitoren, Enzymaktivatoren und pH-Verschiebungen besteht, wobei die Behandlung mit Stressfaktoren die Menge der oxidierten Membran-DNA erhöht und/oder die Demethylierung von Membran-DNA in der Zelle erhöht.

8. Verfahren zum in-vitro-Nachweis von systemischem Lupus erythematodes (SLE), das die folgenden Schritte umfasst:
• In-Kontakt-Bringen einer biologischen Flüssigkeit eines Tieres, die Antikörper enthält, mit dem diagnostischen Mittel gemäß Anspruch 7;
• Messen der Bindung der Antikörper an das diagnostische Mittel; wobei eine Bindung auf eine Krankheit des Tiers hinweist.

9. Verfahren zum in-vitro-Nachweis von systemischem Lupus erythematodes (SLE), das die folgenden Schritte umfasst:
• In-Kontakt-Bringen einer biologischen Probe eines Tieres, die Zellen enthält, mit Antikörpern, die gegen die oxidierte und/oder demethylierte mDNA gemäß Anspruch 4 gerichtet sind;
• Messen der Bindung der Antikörper an die Zellen;
wobei eine Bindung auf eine Krankheit des Tiers hinweist.

10. Verwendung des diagnostischen Mittels gemäß Anspruch 7 zum in-vitro-Nachweis der Bindung von Antikörpern an mDNA.

11. Verwendung gemäß Anspruch 10, wobei die Bindung von Antikörpern, die von einem Tier stammen, auf eine Autoimmunkrankheit des Tiers hinweist.

12. Verfahren zum Screening von Wirkstoffen durch Messen der Bindung von Antikörpern an die mDNA gemäß einem der Ansprüche 4 bis 6 in Gegenwart eines Wirkstoffs.

13. Pharmazeutische Zubereitung, die die mDNA gemäß einem der Ansprüche 4 bis 6 umfasst.

14. Pharmazeutische Zubereitung gemäß Anspruch 13 in Form eines Impfstoffs.

15. Verwendung der mDNA gemäß einem der Ansprüche 4 bis 6 zur Herstellung eines Medikaments zur Behandlung von Autoimmunkrankheiten bei einem Tier.

## Revendications

1. Procédé de préparation d'ADN membranaire (ADNm) oxydé et/ou déméthylé comprenant les étapes consistant à
• traiter une cellule avec un facteur de contrainte choisi dans le groupe constitué par une irradiation aux UV, des réactifs oxydants, des sels de métaux lourds, médicaments, des analogues de nucléoside et nucléotide, des inhibiteurs d'enzyme et une variation de pH
• lyser la cellule pour obtenir un lysat cellulaire
• purifier l'ADNm oxydé et/ou déméthylé à partir du lysat cellulaire.

2. Procédé selon la revendication 1, dans lequel le facteur de contrainte est choisi parmi le dichromate, le peroxyde d'hydrogène, le permanganate, la quinidine, la D-penicillamine, l'hydralazine, le procaïnamide, les métabolites d'ARN/ADN, les analogues de nucléoside et nucléotide tels que la 5-aza-cytidine, la 5-aza-2'-désoxycytidine, les inhibiteurs d'ADN méthylases, les inhibiteurs d'histone désacétylase tels que les butyrates ou la trichostatine A, les inhibiteurs d'histone acétyltransférase, les inhibiteurs d'histone arginine et d'histone lysine méthyltransférases, les inhibiteurs de protéine kinases tels que la staurosporine ou la calphostine C ou K-252A OU H-89, les activateurs de protéine kinases tels que les esters de phorbol ou la bryostatine 1, les inhibiteurs de protéine phosphatases tels que la calyculine A ou l'acide okadaïque, les inhiteurs de poly(ADP-ribose) polymérase tels que le 3-aminobenzamide ou la m-iodobenzylguanidine hémisulfate, les inhibiteurs d'enzymes de conjugaison à l'ubiquitine tels que l'ubiquitine méthylée, des inhibiteurs d'ubiquitine hydrolase C-terminale tels que l'ubiquitine aldéhyde, les inhibiteurs d'enzymes impliquées dans la correction d'erreurs de réplication et/ou de dommage spontané à l'ADN tels que les ions cadmium, des inhibiteurs de la N-glycosylation tels que la tunicamycine, les inhibiteurs de protéasomes 20S ou 26S tels que la lactacystine, les inhibiteurs de farnésylation tels que l'acide alpha-hydroxyfarnésylphosphonique, les inhibiteurs de géranylgéranylation, les inhibiteurs de méthylation de protéine tels que l'ébélactone B, les inhibiteurs de catalase, les inhibiteurs de superoxide dismutase, les inhibiteurs de glutathion péroxidase, les inducteurs d'hyperméthylation d'ADN, les inducteurs de méthylation de phospholipides, les inhibiteurs de méthylation de phospholipides tels que la 3-désazaadénosine ou la 3-désaza-(±)-aristéromycine, ou leurs combinaisons.

3. Procédé selon la revendication 1, dans lequel l'ADNm oxydé et/ou déméthylé est associé à des protéines de liaison à un acide nucléique.

4. ADN membranaire (ADNm) oxydé et/ou déméthylé que l'on peut obtenir selon l'une quelconque des revendications 1 à 3.

5. ADNm selon la revendication 4, associé à des protéines de liaison à l'ADN, en particulier des histones.

6. ADNm selon la revendication 5, dans lequel les protéines de liaison à l'ADN sont hypo- ou hyperacétylées et/ou hypo- ou hyper-phosphorylées, et/ou hypoou hyper-méthylées, et/ou hypo- ou hyper-ubiquitinées, et/ou hypo- ou hyper-(poly-ADP-ribosylées), et/ou hypoou hyper-glycolysées ou leurs combinaisons.

7. Agent de diagnostic comprenant l'ADNm selon l'une quelconque des revendications 4 à 6 ou des cellules que l'on peut obtenir par un procédé permettant de préparer des cellules comprenant de l'ADN membranaire oxydé et/ou déméthylé, comprenant l'étape consistant à traiter une cellule avec un facteur de contrainte choisi dans le groupe constitué par une irradiation aux UV, des réactifs oxydants, des sels de métaux lourds, des médicaments, des analogues de nucléoside et nucléotide, des inhibiteurs d'enzyme, des activateurs d'enzyme et une variation de pH, où le traitement aux facteurs de contrainte augmente la quantité d'ADN membranaire oxydé et/ou augmente la déméthylation de l'ADN membranaire dans la cellule.

8. Procédé pour la détection in vitro du lupus érythémateux disséminé (SLE) comprenant les étapes consistant à
• amener un fluide biologique d'un animal contenant des anticorps en contact avec l'agent de diagnostic selon la revendication 7
• mesurer la liaison desdits anticorps audit agent de diagnostic
où la liaison indique une maladie de l'animal.

9. Procédé pour la détection in vitro du lupus érythémateux disséminé (SLE) comprenant les étapes consistant à
• amener un échantillon biologique d'un animal contenant des cellules en contact avec des anticorps dirigés contre l'ADNm oxydé et/ou déméthylé selon la revendication 4
• mesurer la liaison desdits anticorps auxdites cellules
où la liaison indique une maladie de l'animal.

10. Utilisation de l'agent de diagnostic selon la revendication 7 afin de détecter la liaison des anticorps à l'ADNm in vitro.

11. Utilisation selon la revendication 10, dans laquelle la liaison des anticorps provenant d'un animal indique une maladie auto-immune de l'animal.

12. Procédé permettant de cribler des médicaments en mesurant la liaison des anticorps à l'ADNm selon l'une quelconque des revendications 4 à 6 en présence d'un médicament.

13. Formulation pharmaceutique comprenant l'ADNm selon l'une quelconque des revendications 4 à 6.

14. Formulation pharmaceutique selon la revendication 13 sous forme de vaccin.

15. Utilisation de l'ADNm selon l'une quelconque des revendications 4 à 6 pour la préparation d'un médicament permettant de traiter des maladies auto-immunes chez un animal.
